# EUROPEAN PATENT APPLICATION

(11) **EP 4 775 157 A1**
(43) Date of publication of application: **15.07.2026**
(21) Application number: 23934301.5
(22) Date of filing: 05.09.2023
(51) Int. Cl.: A61B 17/12, A61B 17/34, A61B 17/00

(54) **BENIGN PROSTATIC HYPERPLASIA TREATMENT DEVICE USING ANCHOR ASSEMBLY AND NEEDLE**

(30) Priority: 04.09.2023 KR 20230116635
(71) Applicant: Sorex, Inc., Hanam-si, Gyeonggi-do 12918 (KR)
(72) Inventor: CHUNG, Yoon Ho, Seoul 06057 (KR); PARK, Geon Sun, Seoul 05348 (KR)
(74) Representative: dompatent
(86) International application number: PCT/KR2023/013204
(87) International publication number: WO 2025/053300

(57) **Abstract**

Disclosed is a "benign prostatic hyperplasia surgical device using an anchor assembly and a needle. According to the present invention, the benign prostatic hyperplasia surgical device includes: a plurality of anchors disposed on the prostate gland compressing the urethra; and a ligature thread connecting the plurality of anchors to each other so that the plurality of anchors continuously compress prostatic tissues to allow the urethra to be open. Further, the benign prostatic hyperplasia surgical device of the present invention includes: an elastic needle receiving the plurality of anchors therein and inserted into the urethra to guide the plurality of anchors and the ligature thread so that the plurality of anchors and the ligature thread are located on the prostate gland; and a needle launching means for launching the needle so that the needle is inserted into the urethra to allow the plurality of anchors and the ligature thread to be discharged through the needle.

## Description

### Technical Field

The present invention relates to a benign prostatic hyperplasia surgical device, more particularly to a benign prostatic hyperplasia surgical device that is capable of allowing an anchor assembly to be disposed on the enlarged prostate gland to compress the prostate gland so that the prostatic urethra becomes continuously open.

### Background Art

In general, benign prostatic hyperplasia is a symptom that causes the prostate gland to become enlarged abnormally so that the enlarged prostate gland blocks the prostatic urethra below the bladder, from which urine is excreted, thereby causing urethrophraxis through which urine is not excreted smoothly.

A person who has the benign prostatic hyperplasia experiences great inconveniences in his daily life due to various symptoms, such as frequent urination, urination at night, urgent urination, weak urine stream, feeling of residual urine, and others.

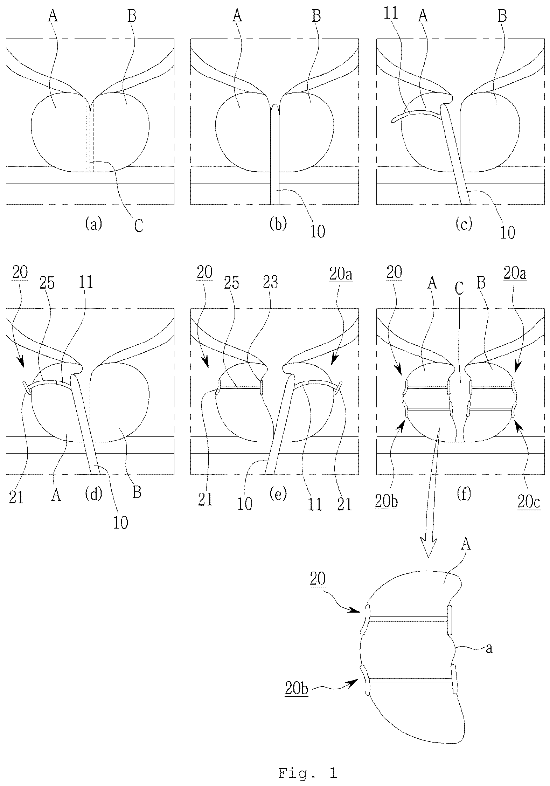

To treat such benign prostatic hyperplasia, a benign prostatic hyperplasia surgical method as shown in FIG. 1 has been carried out.

When the benign prostatic hyperplasia occurs, as shown in FIG. 1a, a pair of prostate lobes A and B is enlarged at both sides of the urethra C and thus blocks the urethra C. As shown in FIG. 1b, a cystoscope 10 is inserted into the urethra by a surgeon, and as shown in FIG. 1c, a medical ligator 11 disposed at an end portion of the cystoscope 10 passes through the prostate lobes A and B. Next, a first implant 20 is put at an upper portion of the left prostate lobe A as shown in FIG. 1d, and a second implant 20a at an upper portion of the right prostate lobe B as shown in FIG. 1e.

After that, as shown in FIG. 1f, a third implant 20b and a fourth implant 20c are put at a lower portion of the left prostate lobe A and a lower portion of the right prostate lobe B.

In this case, the implants 20, 20a, 20b, and 20c have external implants 21 arranged on the outer surface of the prostate lobes A and B and internal implants 23 arranged near the urethra. Each of the external implants 21 and each of the internal implants 23 are connected to each other by a ligature thread 25, and the internal implants 23 compress the prostate lobes A and B outwards by a length of the ligature thread 25, so that the urethra becomes open.

However, such a conventional benign prostatic hyperplasia surgical method has a disadvantage in that the area of the prostatic tissue compressed through one implant 20 is limited to the size of the internal implant 23.

Moreover, there is an interval between the implant 20 arranged at the upper portion of the prostate gland and the implant 20b arranged at the lower portion of the prostate gland, and in this case, the prostate lobe between the internal implant at the upper portion and the internal implant at the lower portion still remains in the enlarged state toward the urethra, without being compressed, so that the prostatic tissue fails to be compressed continuously and only a portion of the prostate gland coming into close contact with the internal implant 23 is compressed.

### Disclosure of the Invention

### Technical Problems

Accordingly, to solve the above-mentioned problems occurring in the related art, it is an object of the present invention to provide a benign prostatic hyperplasia surgical device that is capable of allowing a pair of anchors arranged at upper and lower portions of the prostate gland to be connected to each other by a ligature thread so that a compressed area of the prostate gland is adjusted.

It is another object of the present invention to provide a benign prostatic hyperplasia surgical device that is capable of allowing a ligature thread connecting a pair of anchors to be adjusted in length so that the prostate gland is continuously compressed according to patients' prostate gland sizes.

It is yet another object of the present invention to provide a benign prostatic hyperplasia surgical device that is capable of allowing an anchor assembly to be positioned to various shapes on the prostate gland through an elastic needle and a needle launching means so that the prostate gland is continuously compressed.

### Technical Solutions

The above objects of the present invention are achieved by a benign prostatic hyperplasia surgical device using an anchor assembly and a needle. The benign prostatic hyperplasia surgical device of the present invention may include: a plurality of anchors disposed on the prostate gland compressing the urethra; and a ligature thread connecting the plurality of anchors to each other so that the plurality of anchors continuously compress prostatic tissues to allow the urethra to be open.

Further, the benign prostatic hyperplasia surgical device of the present invention may include: an elastic needle receiving the plurality of anchors therein and inserted into the urethra to guide the plurality of anchors and the ligature thread so that the plurality of anchors and the ligature thread are located on the prostate gland; and a needle launching means for launching the needle so that the needle is inserted into the urethra to allow the plurality of anchors and the ligature thread to be discharged through the needle.

### Advantageous Effects of the Invention

When compared with the conventional surgical method in which a portion of the prostatic tissue is compressed, the benign prostatic hyperplasia surgical device according to the present invention can increase urine flow rate since the prostatic tissue compressed against the ligature thread and the pair of the anchors is continuous, and it is more effective for patients who have a large prostate gland.

Moreover, the benign prostatic hyperplasia surgical device according to the present invention can adjust the length of the ligature thread by winding the free end of the ligature thread, so that compression intensity against the prostate gland can be adjusted to a degree desired by the surgeon.

Therefore, the benign prostatic hyperplasia surgical device according to the present invention can adjust the compression intensity against the prostate gland according to various symptoms of the benign prostatic hyperplasia presented by patient.

The surgical method using the benign prostatic hyperplasia surgical device according to the present invention does not need any general anesthesia or long operation time, unlike laser surgery or electrical surgery using thermal energy in which general anesthesia or long operation time is required.

Additionally, the benign prostatic hyperplasia surgical device according to the present invention can make quick operation time and local anesthesia possible through the simple anchor installation method and have no side effects, such as retrograde ejaculation, impotence or hematuria, since the prostatic tissue is not cut. In addition, the surgical method using the benign prostatic hyperplasia surgical device according to the present invention is minimally invasive, and therefore, if the surgery is ineffective, a patient can have another surgery any time.

### Brief Description of Drawings

FIG. 1 is an exemplary view showing a conventional benign prostatic hyperplasia surgical method.
FIG. 2 is a perspective view showing a benign prostatic hyperplasia surgical device according to an embodiment of the present invention.
FIG. 3 is an exemplary view showing an anchor assembly of the benign prostatic hyperplasia surgical device according to the embodiment of the present invention.
FIG. 4 is an exemplary view showing a state where the anchor assembly of FIG. 3 is mounted on the left prostate lobe.
FIGs. 5 and 6 are exemplary views showing variations of the anchor assembly of the benign prostatic hyperplasia surgical device according to the embodiment of the present invention.
FIGs. 7 and 8 are exemplary views showing states where the variations of the anchor assembly are mounted on the left prostate lobes.
FIG. 9 is an exemplary view showing a process in which the anchor assembly of the present invention is received in a needle and then launched.
FIG. 10 is a sectional view showing the benign prostatic hyperplasia surgical device according to the embodiment of the present invention before a trigger is operated.
FIG. 11 is a sectional view showing the benign prostatic hyperplasia surgical device according to the embodiment of the present invention after the trigger has been pulled.
FIG. 12 is an exploded perspective view showing the benign prostatic hyperplasia surgical device according to the embodiment of the present invention.
FIG. 13 is an exploded perspective view showing a needle guide sheath of the benign prostatic hyperplasia surgical device according to the embodiment of the present invention.
FIG. 14 is an exploded perspective view showing a coupling structure between a needle guide member and a blade of the benign prostatic hyperplasia surgical device according to the embodiment of the present invention.
FIG. 15 is an exemplary view showing an operating process of the blade of the benign prostatic hyperplasia surgical device according to the embodiment of the present invention.
FIG. 16 is a perspective view showing a state where a needle manipulator is coupled to a ligature thread winder in the benign prostatic hyperplasia surgical device according to the embodiment of the present invention.
FIGs. 17 and 18 are exploded perspective views showing the needle manipulator and the ligature thread winder according to the embodiment of the present invention.
FIG. 19 is a side view showing a process of operating the needle manipulator according to the operation of the trigger.
FIG. 20 is a plan view showing the process of operating the needle manipulator according to the operation of the trigger.
FIG. 21 is a sectional view showing the ligature thread winder of the benign prostatic hyperplasia surgical device according to the embodiment of the present invention.
FIG. 22 is an exemplary view showing a process of operating a blade-pressurizing button and a cover of the benign prostatic hyperplasia surgical device according to the embodiment of the present invention.
FIGs. 23 and 24 are exemplary views showing states where the anchor assemblies having various shapes are mounted on the left prostate lobes.

### Mode for Invention

Hereinafter, preferred embodiments of the present invention will now be described in detail with reference to the attached drawings, in which like reference numbers denote corresponding parts throughout the drawings.

The terms "comprising" and "including" in the discussion directed to the present invention and the claims are used in an open-ended fashion and thus should be understood to mean "including", but not limited thereto.

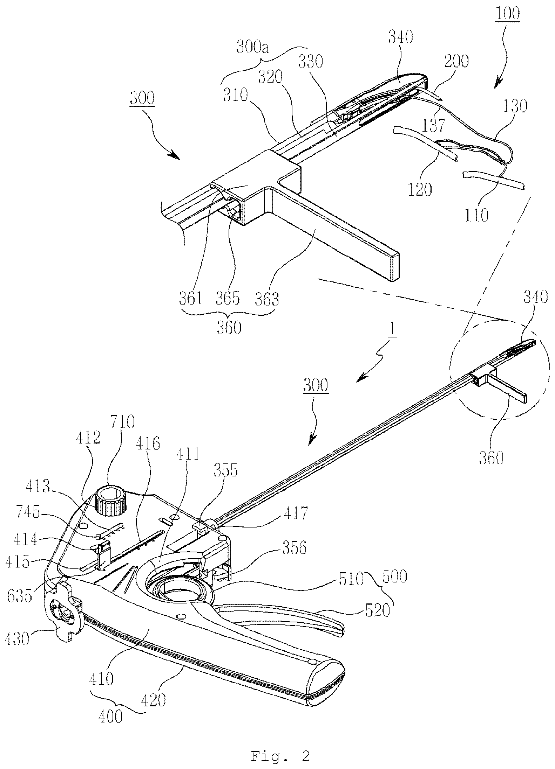

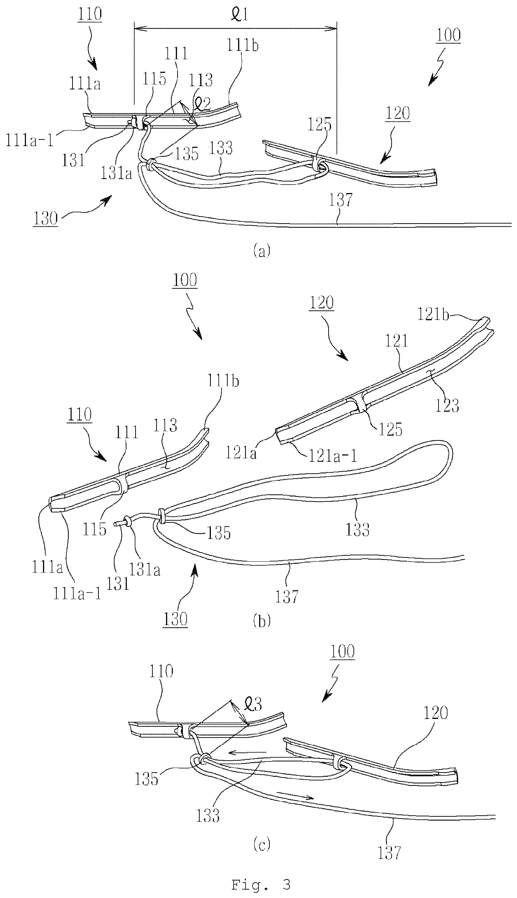

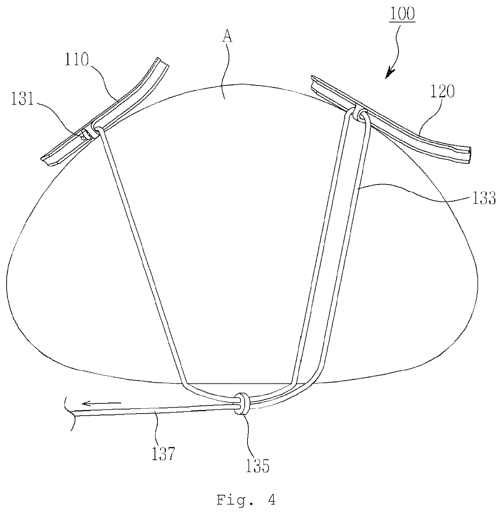

FIG. 2 is a perspective view showing a benign prostatic hyperplasia surgical device according to an embodiment of the present invention, FIG. 3 is an exemplary view showing an anchor assembly of the benign prostatic hyperplasia surgical device according to the embodiment of the present invention, and FIG. 4 is an exemplary view showing a state where the anchor assembly 100 is mounted on the left prostate lobe A.

A benign prostatic hyperplasia surgical device 1 according to an embodiment of the present invention includes an anchor assembly 100 disposed on the outer surface of the prostate gland compressing the prostatic urethra, an elastic needle 200 receiving the anchor assembly 100 therein and inserted into the prostatic urethra to guide the anchor assembly 100 so that the anchor assembly 100 is located on the prostate gland, and a needle launching means for launching the needle 200 so that the anchor assembly 100 is discharged through the needle 200.

In this case, as shown in FIG. 3a, the anchor assembly 100 includes a plurality of anchors 110 and 120 disposed spaced apart from each other on the outer surface of the prostate gland and a ligature thread 130 connecting the anchors 110 and 120 to each other so that the ligature thread 130 continuously compresses the prostatic tissue, thereby ensuring the opening of the prostatic urethra.

The benign prostatic hyperplasia surgical device 1 according to the embodiment of the present invention is configured to allow the anchors 110 and 120 and the ligature thread 130 to be located at desired positions through the needle launching means and to adjust a length of the ligature thread 130 using a ligature thread winder 700 of the needle launching means, so that as shown in FIG. 4, the anchor assembly 100 serves to continuously compress the prostatic tissue at a surgeon's desired compression intensity. Accordingly, the benign prostatic hyperplasia surgical device 1 can adjust a compression length and thickness of the prostate gland according to symptoms of benign prostatic hyperplasia of a patient and increase urine flow rate.

The anchor assembly 100 is located on the outer surface of the prostate gland of a subject and compresses the prostate gland thereagainst so that the urethra is open. A pair of anchor assemblies 100 of the benign prostatic hyperplasia surgical device 1 of the present invention is located on the left prostate lobe A and the right prostate lobe B, thereby completing the surgery for the benign prostatic hyperplasia.

FIG. 3a is a perspective view showing one anchor assembly 100, FIG. 3b is an exploded perspective view showing the anchor assembly 100, FIG. 3c is a perspective view showing a state where a free end 137 of the anchor assembly 100 is pulled, and FIG. 4 is an exemplary view showing a state where the anchor assembly 100 is located on the left prostate lobe A before its length is adjusted.

As shown in FIGs. 3a and 3b, the anchor assembly 100 includes the first anchor 110, the second anchor 120, and the ligature thread 130 connecting the first anchor 110 and the second anchor 120 to each other. The first anchor 110 and the second anchor 120 are different in position from each other and have the same shape as each other, and therefore, only the first anchor 110 will be explained in detail.

The first anchor 110 includes a first anchor body 111 having the shape of a tube with a semicircular sectional area and a first thread-coupling loop 115 protruding from the middle region of the first anchor body 111 in such a way as to couple the ligature thread 130 thereto.

Further, the first anchor 110 includes a first thread-receiving groove 113 concavely formed inside the first anchor body 111 in a longitudinal direction thereof to receive the ligature thread 130 therein. The first anchor body 111 is made of a metal or a metal alloy. Desirably, the first anchor body 111 is made of nitinol. A horizontal end 111a is formed on one end of the first anchor body 111, and a bent end 111b with a given curvature on the other end thereof. As shown in FIG. 4, the formation of the bent end 111b on the other end of the first anchor body 111 enables the first anchor 110 or the second anchor 120 to be stably fixed in position when it is mounted on the prostate lobe A or B.

Through the formation of the bent end 111b, the first anchor 110, which passes through the prostatic tissue and is thus located on the outer surface of the prostate gland, while being bent relative to the outer surface of the prostate gland, is thus prevented from moving back into the through hole in the prostate gland formed by the needle 200.

That is, as shown in FIG. 4, if the needle 200 penetrates the prostate lobe A to discharge the first anchor 110 to the outer side of the prostate lobe A, during the surgical process, the first anchor 110 is bent as the bent end 111b is elastically bent. The bent first anchor 110 is caught by the hole in the prostate lobe A formed by the needle 200 and thus prevented from moving backward, so that it is stably seated onto the outer surface of the prostate lobe A.

The first anchor 110 has a slant contact end 111a-1 formed slantly on the horizontal end 111a so that the first anchor 110 comes into close contact with the outer surface of the prostate lobe A correspondingly to the curvature of the outer surface of the prostate lobe A or B.

The first thread-coupling loop 115, to which the ligature thread 130 inserted into the first thread-receiving groove 113 is knotted or tied, allows the ligature thread 130 to be fixed in position. The first thread-coupling loop 115 serves to allow the first anchor 110 to be kept fixed to the ligature thread 130.

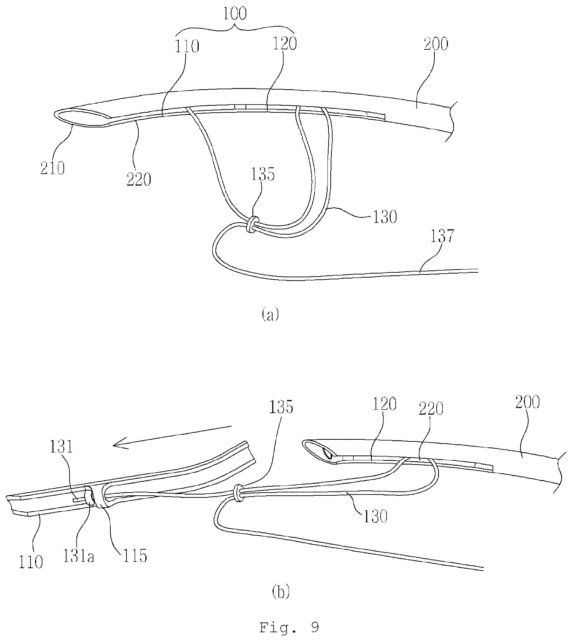

The first anchor 110 and the second anchor 120 are bent by the bent ends 111b and 121b, but as shown in FIG. 9a, if they are received in the needle 200, they are elastically deformed according to the shape of the needle 200. If the first anchor 110 and the second anchor 120 are discharged from the needle 200, as shown in FIG. 9b, the bent ends 111b and 121b are elastically returned to their original bent state.

The ligature thread 130 connects the first anchor 110 and the second anchor 120 to each other in such a way as to be adjustable in length. In this case, the prostate gland is not compressed at specific portions against the first anchor 110 and the second anchor 120, independently of each other, but the prostatic tissue is continuously compressed against an arch or "⊏"-shaped line formed by connecting the first anchor 110, the second anchor 120, and the ligature thread 130.

As shown in FIG. 3b, the ligature thread 130 includes a fixed end 131 coupled to the first anchor 110, a length-adjustable loop 133 coupled to the second anchor 120, a free end 137 connected to an end portion of the length-adjustable loop 133, and a slip knot 135 for tying the free end 137 and the length-adjustable loop 133.

The fixed end 131 is inserted into the first thread-coupling loop 115 of the first anchor 110 and thus forms a fixing knot 131a thereon. The fixing knot 131a has a height higher than the first thread-coupling loop 115 so that the fixed end 131 is caught on the first thread-coupling loop 115 and prevented from moving to the other end of the first thread-coupling loop 115 to one side.

The length-adjustable loop 133 enters the second thread-coupling loop 125 of the second anchor 120 and is then discharged to the opposite side to the side where it enters, so that it takes the shape of a loop. The length-adjustable loop 133 is formed between the fixed end 131 and the free end 137 by means of the slip knot 135.

If the free end 137 is pulled, as shown in FIG. 3c, the length-adjustable loop 133 gets shorter in length because the first anchor 110 coupled to the fixed end 131 is fixed in position, so that the prostatic tissue surrounded with the first anchor 110 and the length-adjustable loop 133 becomes compressed.

If the first anchor 110 and the second anchor 120 are located on the outer side of the prostate lobe A, as shown in FIG. 4, the ligature thread 130 is in a loose state. In the above state, if the ligature thread 130 is pulled by the surgeon's manipulation of the needle launching means, the free end 137 gets shorter in length and the length-adjustable loop 133 also gets shorter in length, thereby resulting in the compression of the prostate lobe A.

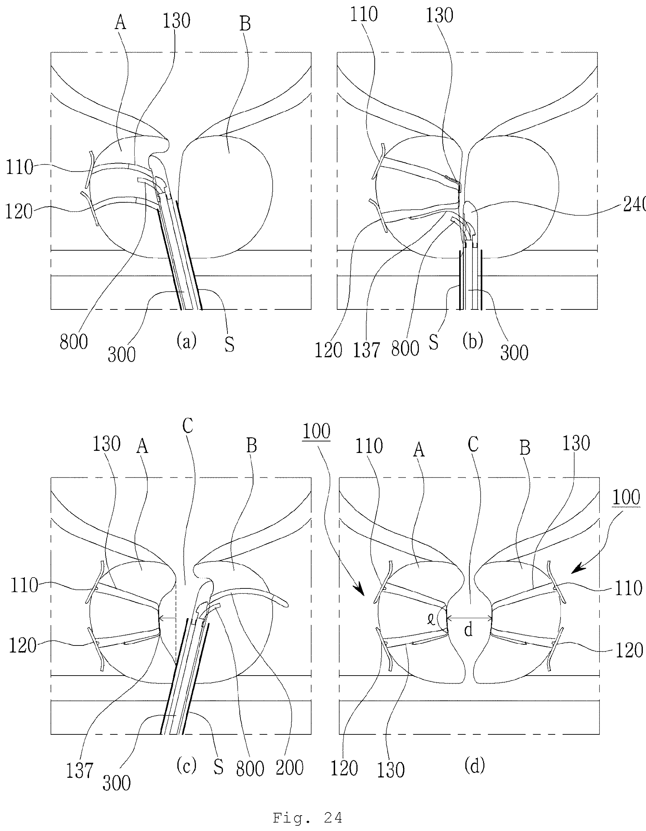

That is, as shown in FIG. 24b, if the first anchor 110 and the second anchor 120 are located on the outer side of the prostate gland, the ligature thread 130 is connected loosely. In the above state, if the free end 137 is pulled to tighten the ligature thread 130 by the surgeon's manipulation of the needle launching means, as shown in FIG. 24c, the first anchor 110, the second anchor 120, and the ligature thread 130 continuously compress the prostate lobe A, while surrounding the prostate lobe A. Since the first anchor 110 and the second anchor 120 are connected to each other by means of the ligature thread 130, a space portion between the first anchor 110 and the second anchor 120 is also continuously compressed so that the prostatic urethra C is opened.

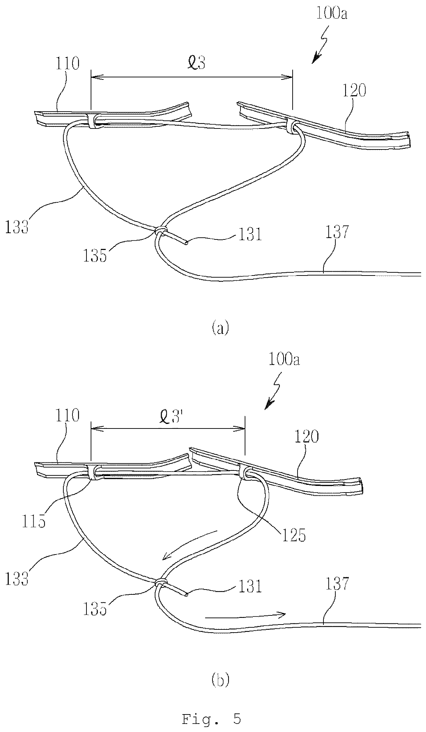

FIG. 5a is a perspective view showing an anchor assembly 100a according to a first variation of the present invention, and FIG. 5b is an exemplary view showing a state where a free end of the anchor assembly 100a according to the first variation of the present invention is pulled.

The anchor assembly 100 as mentioned above according to the embodiment of the present invention is configured to allow the fixed end 131 of the ligature thread 130 to be coupled to the first anchor 110, while allowing the length-adjustable loop 133 to be coupled to the second anchor 120.

Contrarily, the anchor assembly 100a according to the first variation of the present invention is configured to allow both of a first anchor 110 and a second anchor 120 to be coupled to a length-adjustable loop 133, so that if a free end 137 is pulled, both of the first anchor 110 and the second anchor 120 can move.

As shown in FIG. 3a, the anchor assembly 100 according to the embodiment of the present invention is configured to allow a length ℓ2 between the fixed end 131 and the slip knot 135 to be fixed, without any change, because the fixed end 131 is fixed to the first anchor 110. Therefore, as shown in FIG. 3c, even if the free end 137 is pulled, a distance ℓ1 between the first anchor 110 and the second anchor 120 cannot be reduced until they meet.

Contrarily, the anchor assembly 100a according to the first variation of the present invention is configured to allow a distance ℓ3 between the first anchor 110 and the second anchor 120 to be reduced to a distance ℓ3' where the first anchor 110 and the second anchor 120 have a contact with each other if the free end 137 is pulled, as shown in FIG. 5b. As a result, advantageously, a distance between the first anchor 110 and the second anchor 120 can be easily adjusted by a surgeon.

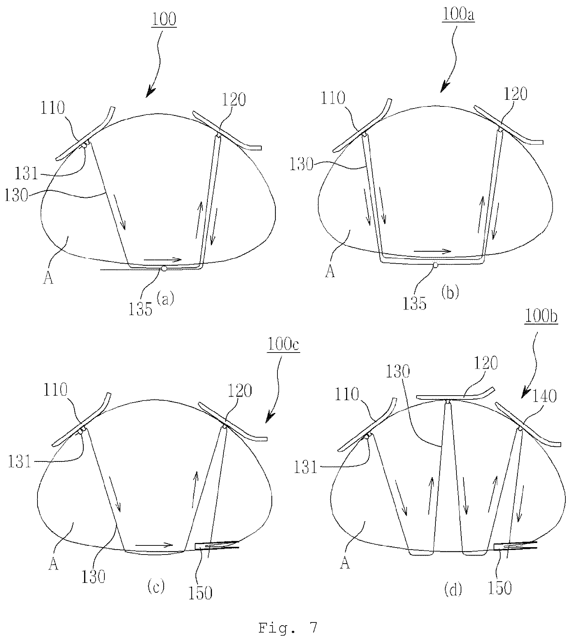

FIG. 7b is an exemplary view showing a state where the anchor assembly 100a according to the first variation of the present invention is mounted on the prostate lobe A or B. As shown, the first anchor 110 and the second anchor 120 are coupled to the outer peripheral surface of the prostate lobe A or B, and the ligature thread 130 is located on the prostatic urethra, so that the prostatic urethra is opened.

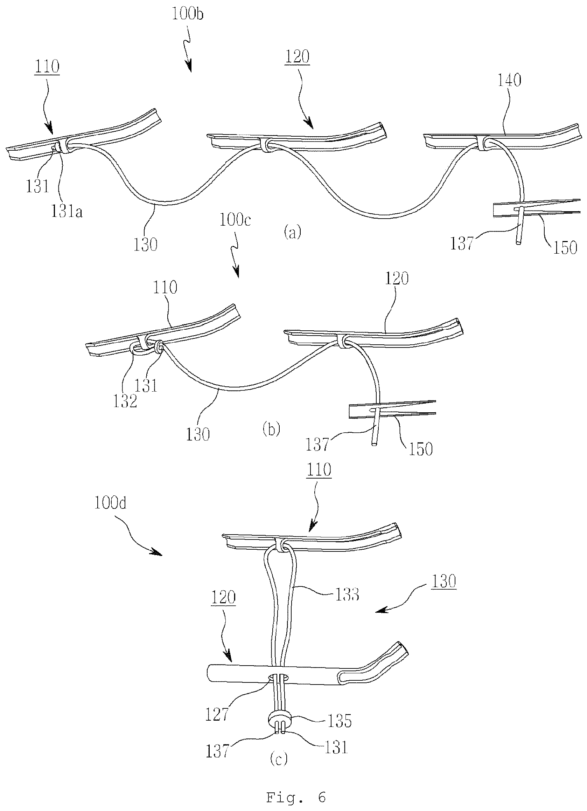

Further, FIG. 6a is an exemplary view showing an anchor assembly 100b according to a second variation of the present invention. As shown, the anchor assembly 100b according to the second variation of the present invention includes a first anchor 110, a second anchor 120, and a third anchor 140. The anchor assembly 100b according to the second variation of the present invention is configured to allow three anchors 110, 120, and 140 to be coupled to a ligature thread 130 in such a way as to be spaced apart from one another by a given distance, while allowing a lock anchor 150 to be coupled to a free end 137 of the ligature thread 130.

A fixed end 131 is fixed to the first anchor 110 with a fixing knot 131a, and the second anchor 120 and the third anchor 140 are coupled to the path of the ligature thread 130, sequentially. FIG. 7d is an exemplary view showing a state where the anchor assembly 100b according to the second variation of the present invention is mounted on the prostate lobe A or B.

As shown, the three anchors 110, 120, and 140 are coupled to the outer peripheral surface of the prostate lobe A or B, sequentially, and the ligature thread 130 penetrates the prostate lobe A or B and connects the three anchors 110, 120, and 140 to one another. Further, the lock anchor 150 is located on a portion of the prostatic urethra facing the third anchor 140 and fixes the position of the free end 137 thereto.

The lock anchor 150 is made of a plate-shaped material and has a "V"-shaped thread guide groove formed on the front end thereof. As a result, the free end is fitted to the thread guide groove of the lock anchor 150 that becomes narrow in its width and thus fixed in position.

The lock anchor 150 may be freely changed in shape only if it fixes the position of the free end 137.

FIG. 6b is an exemplary view showing an anchor assembly 100c according to a third variation of the present invention. The anchor assembly 100c according to the third variation of the present invention is configured to allow a first anchor 110 and a second anchor 120 to be coupled to a ligature thread 130 sequentially, without having any length-adjustable loop 133, while having a lock anchor 150 on the end of the ligature thread 130. Further, the first anchor 110 is fixed to a fixed end 131 and a fixed loop 132 of the ligature thread 130.

In this case, as shown in FIG. 7c, the anchor assembly 100c according to the third variation of the present invention is mounted on the prostate lobe A or B. As shown in the second and third variations of the present invention, if the plurality of anchors 110 and 120 are coupled to the ligature thread 130 sequentially, without any length-adjustable loop 133, the free end 137 is fixed in position by means of the lock anchor 150. The length-adjustable loop 133 is adjusted in length by means of the slip knot 135 and thus fixed to the prostate lobe A, but if the length-adjustable loop 133 does not exist, the free end 137 is fitted to the thread guide groove of the lock anchor 150 and thus fixed in position.

Further, FIG. 6c is an exemplary view showing an anchor assembly 100d according to a fourth variation of the present invention that is configured to allow a first anchor 110 to be located to face a second anchor 120. A length-adjustable loop 133 of a ligature thread 130 is coupled to the first anchor 110, and next, it is inserted into a ligature thread-passing bridge 127 of the second anchor 120 and then coupled to a slip knot 135 below the second anchor 120. A free end 137 and a fixed end 131 are coupled to the slip knot 135, together.

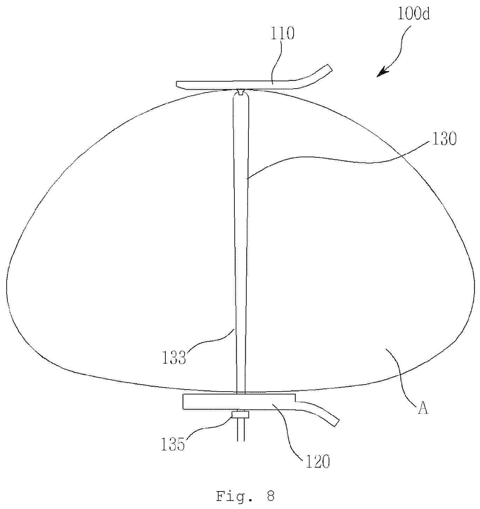

As shown in FIG. 8, the anchor assembly 100d according to the fourth variation of the present invention is configured to allow the first anchor 110 and the second anchor 120 to be located to face each other on the outer and inner sides of the prostate lobe A or B. The anchor assembly 100d is different from the anchor assemblies 100, 100a, 100b, and 100c as mentioned above where both of the anchors 110 and 120 are located on the outer side of the prostate lobe A or B.

If the free end 137 is pulled and thus decreased in length by means of the surgeon, the length-adjustable loop 133 gets shorter in length so that the prostate lobe A or B is compressed against the second anchor 120, thereby resulting in the opening of the prostatic urethra.

In this case, the slip knot 135 is located below the second anchor 120, but in some cases, the slip knot 135 may be located between the first anchor 110 and the second anchor 120.

The anchor assembly 100 according to the embodiment of the present invention is inserted into the prostate lobe A or B in a state of being received in the elastic needle 200, and next, the anchor assembly 100 is discharged to the outside from the needle 200 by means of the manipulation of the needle launching means and thus located on the outer side of the prostate lobe A or B.

FIG. 9a is an exemplary view showing a state where the anchor assembly 100 is received in the needle 200, and FIG. 9b is an exemplary view showing a state where the first anchor 110 is discharged from the needle 200.

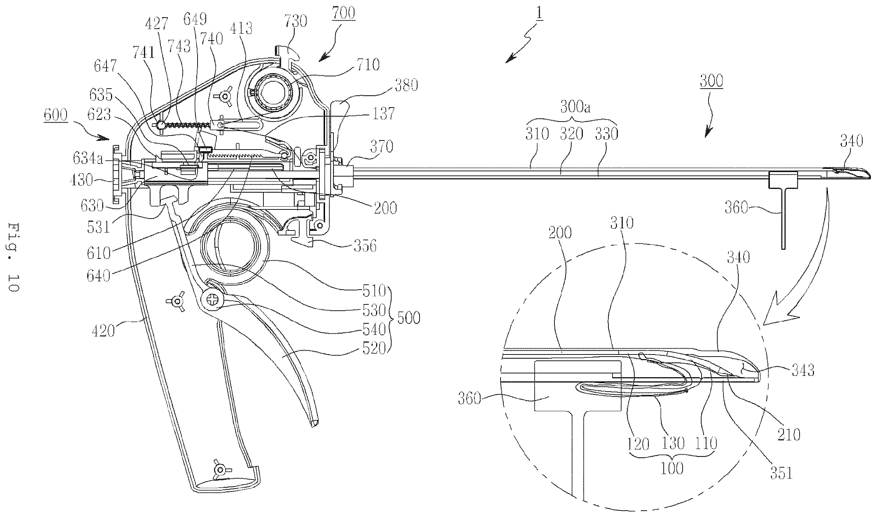

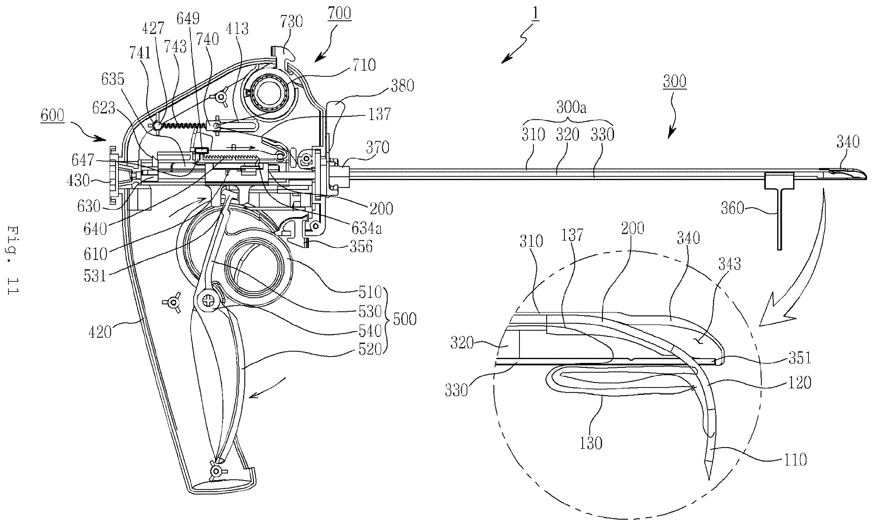

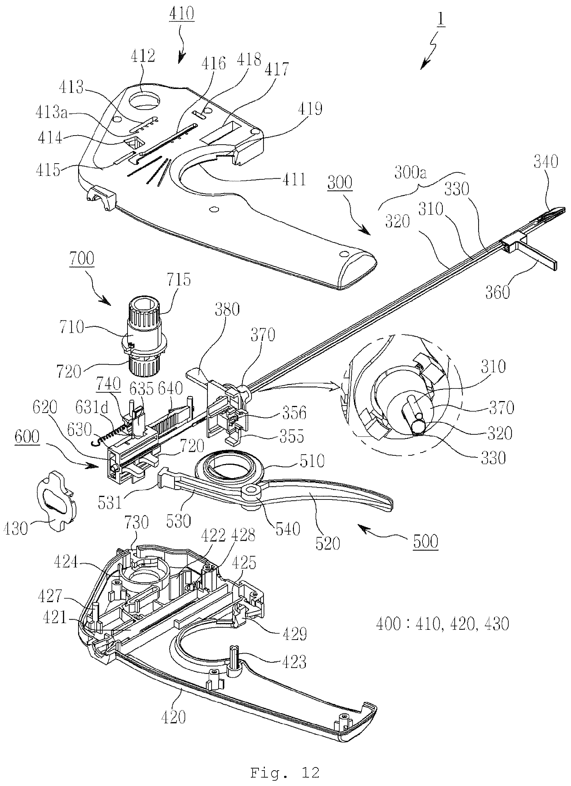

FIG. 10 is a sectional view showing a state before the needle 200 is launched from the needle launching means, FIG. 11 is a sectional view showing a state where the needle 200 is launched from the needle launching means, and FIG. 12 is an exploded perspective view showing the needle launching means.

The needle launching means includes all components of the benign prostatic hyperplasia surgical device 1 except the anchor assembly 100 and the needle 200. The needle launching means may be freely changed in shape only if it launches the anchor assembly 100 and the needle 200 by means of the manipulation of the surgeon and locates them on the prostate gland.

The needle launching means according to the embodiment of the present invention includes a needle-receiving sheath 300 adapted to receive the needle 200 therein, be inserted into the urethra, and guide the needle 200 so that the anchor assembly 200 is located on the prostate gland, a needle manipulator 600 for manipulating the movement of the needle 200 so that the needle 200 is discharged through the front end of the needle-receiving sheath 300, a ligature thread winder 700 located on one side of the needle manipulator 600 to wind the free end 137 of the ligature thread 130 thereon so that compression intensity of the anchor assembly 100 against the prostate gland is adjusted, a handle frame 400 adapted to receive the rear end of the needle-receiving sheath 300, the needle manipulator 600, and the ligature thread winder 700 therein and held by the surgeon's hand, and a trigger 500 coupled to the handle frame 400 to allow the launching of the needle 200 to be manipulated by the surgeon.

The needle 200 has the shape of a tube with a given length, and as shown in FIG. 10, the needle 200 is inserted into a needle insertion tube 310 of the needle-receiving sheath 300. If the trigger 500 is manipulated by the surgeon in the state where the needle 200 is inserted into the needle-receiving sheath 300, the needle 200 is bent to a given angle along a needle guide member 340 and then inserted into the prostate lobe A or B.

The needle 200 is made of a metal or metal alloy, preferably nitinol. The needle 200 is made to be bent on the front end thereof, and if the needle 200 is received in the needle insertion tube 310, as shown by an enlarged portion of FIG. 10, the needle 200 is kept in the shape bent elastically by means of the curvature of the needle guide member 340.

If the needle 200 is launched by the trigger 500 and thus discharged to the outside from the needle guide member 340, as shown by an enlarged portion of FIG. 11, the needle 200 is discharged to be adjusted in angle by means of the needle guide member 340 and then bent correspondingly to the curvature when manufactured.

The needle 200 has a sharp front end 210 so that it can be easily inserted into the prostate lobe A or B. Further, the needle 200 has a needle slot 220 incised from the front end 210 to a given portion of an inside thereof. The needle slot 220 serves to allow the ligature thread 130 of the anchor assembly 100 received in the needle 200 to be exposed to the outside.

A length of the needle slot 220 is equal to or shorter than a length obtained by adding the lengths of the first anchor 110 and the second anchor 120, thereby preventing the ligature thread 130 from interfering with the launching of the first anchor 110 and the second anchor 120.

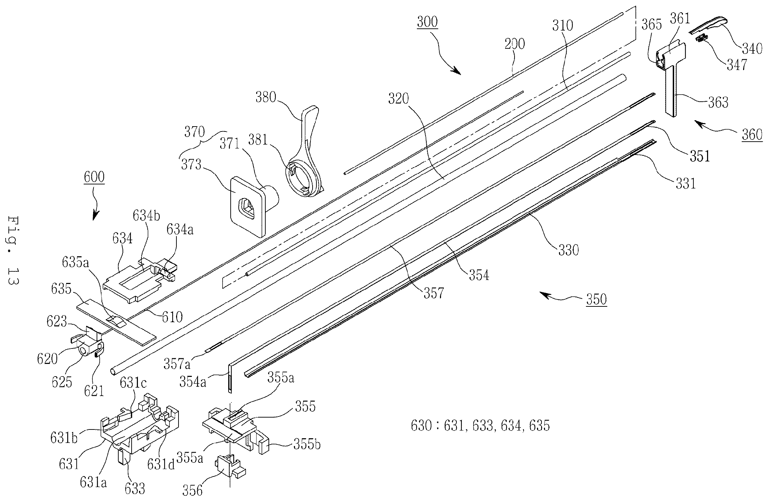

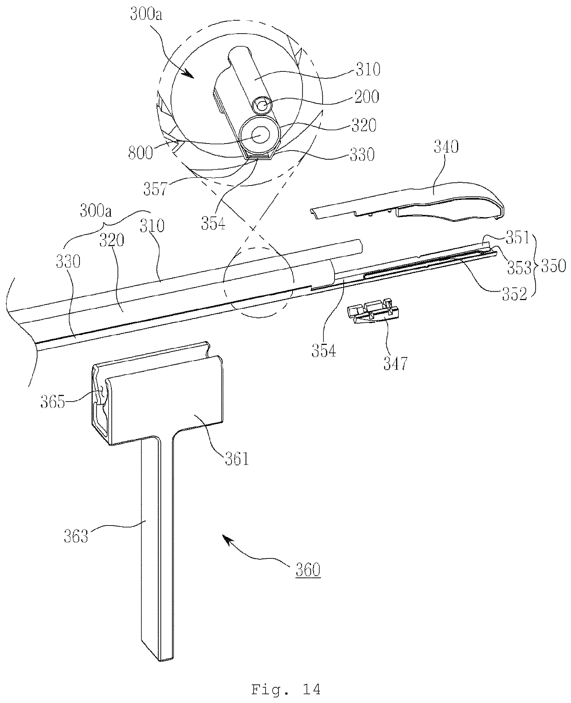

FIG. 13 is an exploded perspective view showing a coupling structure between the needle-receiving sheath 300 and the needle manipulator 600, and FIG. 14 is an exemplary view showing a coupling structure among a sheath body 300a, the needle guide member 340, and a suture arranger 360.

The needle-receiving sheath 300 receives the needle 200 and a urethroscope 800 therein and guides the anchor assembly 100 to the prostate lobe A or B through the needle 200, so that the anchor assembly 100 is fixed in position. The needle-receiving sheath 300 has a rear end coupled to the handle frame 400 and a front end extending from the handle frame 400 in such a way as to be inserted into the prostatic urethra C through an outer sheath S.

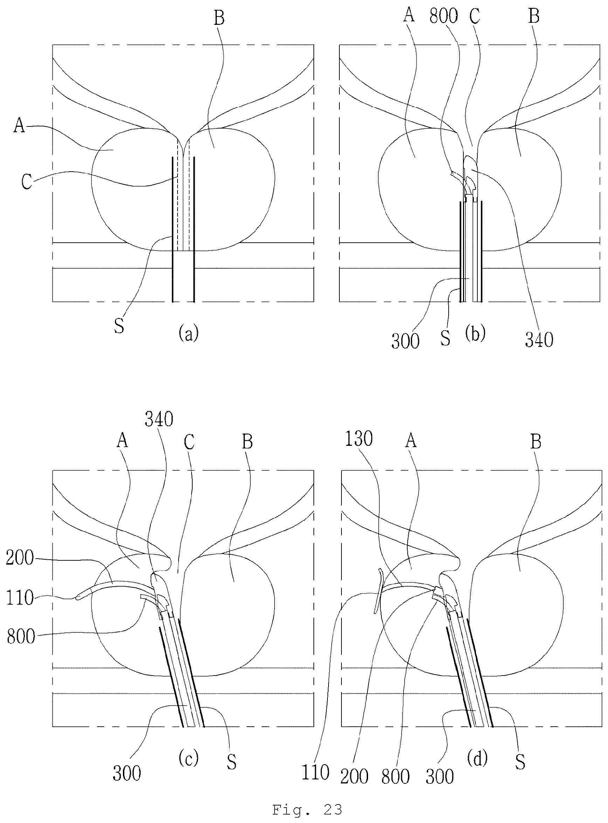

As shown in FIGs. 23 and 24, the urethroscope 800 is inserted by the surgeon into the needle-receiving sheath 300 inserted into the prostatic urethra C through the outer sheath S to inspect the interior of the prostate gland, and then, the needle 200 penetrates the prostate lobe A or B to allow the pair of anchors 110 and 120 to be located on the outer side of the prostate lobe A or B.

As shown in FIGs. 12 and 13, the needle-receiving sheath 300 includes a sheath body 300a made by integrally coupling the needle insertion tube 310, a urethroscope insertion tube 320, and a blade shaft-moving tube 330 with one another, the needle guide member 340 disposed on the front end of the sheath body 300a to guide the needle 200 to the prostate lobe A or B, a blade 350 moving forward and backward inside the blade shaft-moving tube 330 to cut the free end 137 of the ligature thread 130, a suture arranger 360 for organizing the ligature thread 130 exposed to the outside from the needle 200, a sheath cylinder 370 for coupling the sheath body 300a to the handle frame 400, and a sheath lock button 380 for fixing the outer sheath S to the sheath cylinder 370.

The needle insertion tube 310 receives the needle 200 therein, and the urethroscope insertion tube 320 is coupled integrally with the underside of the needle insertion tube 310 and receives the urethroscope 800 therein. The blade shaft-moving tube 330 is coupled integrally with the underside of the urethroscope insertion tube 320 and receives a blade plate connection shaft 354 and a blade support plate 357 therein in such a way as to be movable therein.

As shown by an enlarged portion of FIG. 14, the needle 200 is received inside the needle insertion tube 310, the urethroscope 800 inside the urethroscope insertion tube 320, and the blade plate connection shaft 354 and the blade support plate 357 inside the blade shaft-moving tube 330. The needle insertion tube 310 and the urethroscope insertion tube 320 are hollow tubes, and the blade shaft-moving tube 330 has a trapezoidal sectional area and is fixedly coupled to the underside of the urethroscope insertion tube 320.

The sheath body 300a is configured to allow the rear end thereof to be inserted into the handle frame 400 by means of the sheath cylinder 370 and the sheath lock button 380. In this case, as shown in FIG. 13, the needle 200 is inserted into the needle-receiving tube 310, and a pusher 610 of the needle manipulator 600 is inserted into the rear side of the needle 200. The pusher 610 is fixedly coupled to a pusher holder 620, and through the forward and backward movements of the pusher holder 620 by the surgeon, the pusher 610 moves forward and backward inside the needle 200 so that the anchor assembly 100 is manipulated to be discharged.

The urethroscope insertion tube 320 is longer than the needle insertion tube 310 and the blade shaft-moving tube 330, and the rear end of the urethroscope insertion tube 320 passes through the pusher holder 620. The urethroscope 800, which is inserted through an urethroscope-fixing member 430, is inserted into the urethroscope insertion tube 320.

The needle guide member 340 is detachably coupled to the front ends of the needle insertion tube 310 and the blade shaft-moving tube 330.

In this case, as shown in FIG. 1, the benign prostatic hyperplasia surgical device 1 according to the embodiment of the present invention is provided with the components completely put together, then sterilized and packed, and finally provided to a hospital. Accordingly, the surgeon does not need to couple or separate the respective components of the benign prostatic hyperplasia surgical device 1, so that he or she just manipulates the corresponding components as put together, thereby improving the conveniences of use.

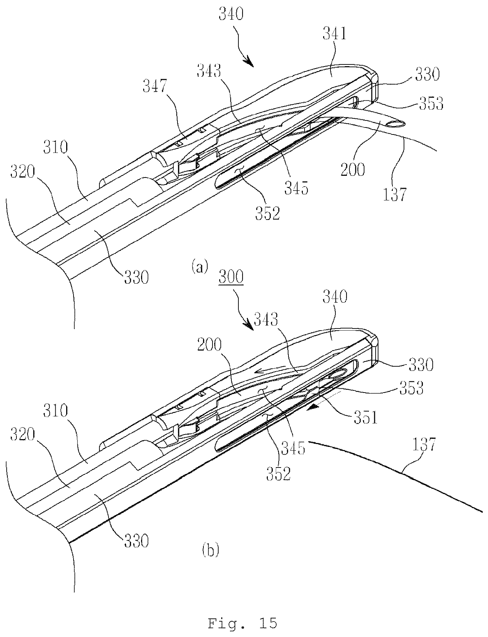

FIG. 15 is an exemplary view showing the operating process of the needle guide member 340 and the blade 350. The needle guide member 340 includes a guide body 341 and a sheath-fixing member 347 for coupling the guide body 341 to the needle insertion tube 310.

The needle guide member 340 is coupled to the needle insertion tube 310 as the sheath-fixing member 347 fixes a top of the needle insertion tube 310 to a top of the guide body 341. The needle guide member 340 has a needle exposure hole 345 formed between the sheath-fixing member 347 and the blade shaft-moving tube 330 to expose the needle 200 to the outside. Through the needle exposure hole 345, the urethroscope 800 moving to the urethroscope insertion tube 320 is exposed to the inside of the prostatic urethra C.

Further, as shown by the enlarged portion of FIG. 10, the ligature thread 130 received in the needle 200 is exposed to the outside through the needle exposure hole 345 and moves to the prostate lobe A or B, without any interruption.

The needle guide member 340 has a needle-guiding curved surface 343 formed on the inner wall of the upper portion of the guide body 341 forming the needle exposure hole 345 and serves to guide the needle 200 to the urethra C. The needle 200 is located inside the needle insertion tube 310 in an initial state, as shown in FIG. 10, and if the trigger 500 is manipulated by the surgeon, as shown in FIG. 11, the needle 200 is exposed to the outside by a given length through the needle guide member 340.

In this case, the needle 200 is bent to a given angle along the needle-guiding curved surface 343 and penetrates the prostate lobe A or B.

The blade 350 is movably located inside the blade shaft-moving tube 330 and is manipulated by the surgeon to cut the ligature thread 130 after the first anchor 110 and the second anchor 120 have been located on the prostate lobe A or B.

As shown in FIG. 13, the blade 350 includes the blade plate connection shaft 354 with a blade plate 351 and the blade support plate 357 fixedly coupled to a top of the blade plate connection shaft 354 to support the blade plate connection shaft 354.

A vertical coupling end 354a formed on the rear end of blade plate connection shaft 354 is coupled to a blade-pressurizing button 355, and the blade-pressurizing button 355 is moved forward and backward by the surgeon to cut the ligature thread 130.

The blade shaft-moving tube 330 has an external needle exposure hole 331 formed on the front end thereof, and the blade plate 351 has an internal needle exposure hole 352. As shown in FIG. 15a, the external needle exposure hole 331 is overlaid on the internal needle exposure hole 352, and the needle 200 is discharged through the external needle exposure hole 331 to allow the pair of anchors 110 and 120 and the ligature thread 130 to be located on the prostate lobe A or B.

The blade plate 351 has a blade slit 353. The blade slit 353 becomes narrow in width and has sharp inner end portions facing each other. If the blade-pressurizing button 355 is pulled backward by the surgeon to pull the blade plate 351, as shown in FIG. 15b, the free end 137 of the ligature thread 130 is fitted to the blade slit 353 and thus cut.

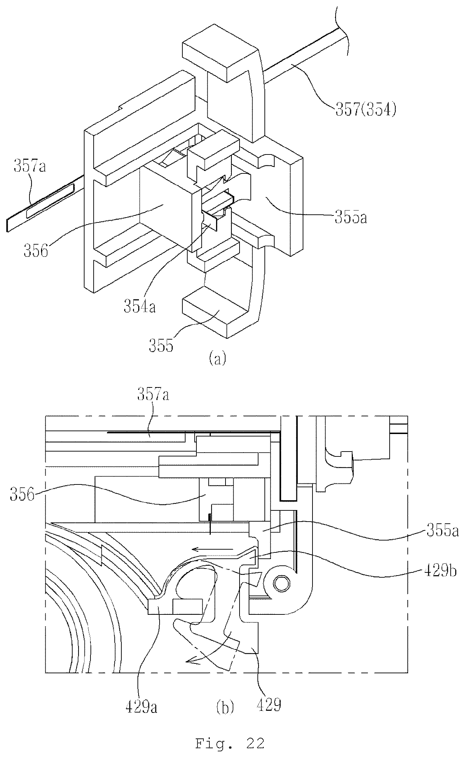

The blade-pressurizing button 355 and a cover 356 are used by the surgeon to cut the free end 137 of the ligature thread 130. FIG. 22a is an exemplary view showing a state where the blade-pressurizing button 355 and the cover 356 are coupled to the vertical coupling end 354a, and FIG. 22b is an exemplary view showing an operation of a blade lock releasing lever 429.

As shown, the vertical coupling end 354a is fitted to the blade-pressurizing button 355, so that the blade 350 is fixed in position. The cover 356 is fitted to the blade-pressurizing button 355, so that the vertical coupling end 354a is fixed in position.

The blade-pressurizing button 355 has a locking wing 355a protruding downward from the rear side thereof. The locking wing 355a comes into close contact with the blade lock releasing lever 429 of the handle frame 400 and thus fixes the blade lock releasing lever 429 in position. If the surgeon desires to cut the free end 137, the blade lock releasing lever 429 is pulled in a direction of an arrow to release the lock state with the locking wing 355a, and next, the blade-pressurizing button 355 is pulled backward.

Further, as shown in FIG. 10, the suture arranger 360 organizes the ligature thread 130 to prevent the ligature thread 130 complicatedly exposed to the outside from the external needle exposure hole 331 from interfering with the discharge path of the needle 200.

As shown in FIG. 14, the suture arranger 360 includes an arranger body 361 open on a top thereof in such a way as to be fitted to the sheath body 300a, a handle 363 extending vertically from an underside of the arranger body 361, and a sheath-receiving path 365 formed inside the arranger body 361.

In an initial packed state of the benign prostatic hyperplasia surgical device 1, as shown in FIG. 10, the suture arranger 360 is coupled to the front end of the sheath body 300a, and the ligature thread 130 is organizedly received inside the sheath body 300a.

If the surgeon is ready to locate the anchor assembly 100 on the prostate lobe A or B, he or she holds the handle 363 to pull down the arranger body 361 so that the suture arranger 360 is separated from the sheath body 300a.

The sheath cylinder 370 fixes the sheath body 300a and the handle frame 400 thereto. As shown in FIG. 12, a right frame 410 and a left frame 420 of the handle frame 400 have sheath-coupling member insertion grooves 425 on the front ends thereof, and as shown in FIG. 10, the sheath cylinder 370 to which the sheath body 300a is fitted is coupled to the handle frame 400.

In the above state, the sheath lock button 380 is fitted to the front end of the sheath cylinder 370 to fix the sheath body 300a to the outer sheath S. As shown in FIG. 13, the sheath cylinder 370 includes a sheath coupling tube 371 for receiving the sheath body 300a therein and a frame coupling plate 373 extending vertically from the rear end of the sheath coupling tube 371. The frame coupling plate 373 is fitted to the sheath coupling member insertion grooves 425 of the right frame 410 and the left frame 420.

The sheath lock button 380 has fixing protrusions 381 protruding from the inside thereof in such a way as to be fittedly coupled to the sheath coupling tube 371, so that it is fixed in position.

As shown in FIG. 2 or FIGs. 12 and 13, the handle frame 400 is coupled to the rear side of the needle-receiving sheath 300 and receives the trigger 500, the needle manipulator 600, and the ligature thread winder 700 therein. In a state where the handle frame 400 is held by the surgeon's hand, the needle-receiving sheath 300 is inserted into the prostatic urethra C by the manipulation of the needle-receiving sheath 300 and the needle manipulator 600, and next, the needle 200 and the anchor assembly 100 are adjusted in position to locate the anchor assembly 100 on the prostate gland.

The handle frame 400 is configured to allow the left frame 420 and the right frame 410 to be detachably coupled to each other. The left frame 420 and the right frame 410 have the shapes of guns held by a hand.

The left frame 420 and the right frame 410 are symmetrically shaped, and each of them will be explained with reference to FIG. 12.

The right frame 410 has a trigger loop-receiving groove 411 formed on a lower portion thereof to receive a loop 510 of the trigger 500. Further, the right frame 410 has a thread winder-receiving groove 412, an indicator-receiving groove 413, a pusher lever button-receiving groove 414, a manually pusher holder-operating slit 415, a pusher block-moving slit 416, a blade-pressurizing button-moving slit 417, and a manually ligature thread-cutting slit 418 formed on the upper portion thereof.

The left frame 420 has a needle manipulator-receiving groove 421, a trigger-rotating shaft 423, a lock releasing button-locking end 422, the sheath coupling member insertion groove 425, an indicator coupling shaft 427, a pusher lever-rotating shaft coupling tube 428, and the blade lock releasing lever 429 formed at the inside thereof.

The urethroscope-fixing member 430 is detachably coupled to the rear sides of the left frame 420 and the right frame 410. The urethroscope 800 is inserted into the urethroscope insertion tube 320 by means of the urethroscope-fixing member 430.

As shown in FIG. 2, a winder shaft 710 of the ligature thread winder 700 is rotatably inserted into the thread winder-receiving groove 412 of the right frame 410 so that the ligature thread 130 wound thereon is wound or unwound, and the indicator-receiving groove 413 is provided to allow a thread indicator 740 of the ligature thread winder 700 to be movably coupled thereto in forward and backward directions so that a current degree of pulling of the free end 137 of the ligature thread 130 is indicated on the outside. Further, scales 413a are made below the indicator-receiving groove 413 in a longitudinal direction of the indicator-receiving groove 413, so that the current degree of pulling of the free end 137 can be checked accurately by the surgeon.

The pusher lever button-receiving groove 414 is provided to allow a pusher lever button 649 of a pusher lever 640 to be rotatable upward and downward. Before a needle holder 630 moves forward, the pusher lever button 649 exposed to the outside from the pusher lever button-receiving groove 414 of the right frame 410 rotates upward by the surgeon. Operations related thereto will be explained in detail later.

The manually pusher holder-operating slit 415 is a slit formed to pass through the right frame 410 to allow the pusher holder 620 to be pushed manually by the surgeon in a state where the needle 200 is not launched. The pusher block-moving slit 416 is adapted to insert both ends of a pusher block 635 of the needle holder 630 thereinto. The pusher block 635 protrudingly exposed to the outside from both sides of the pusher block-moving slit 416 is pressurized by the surgeon's hand so that the pusher block 635 is adjusted in position.

The blade-pressurizing button-moving slit 417 is adapted to receive both ends of the blade-pressurizing button 355 therein. The blade-pressurizing button 355 exposed to the outside from the blade-pressurizing button-moving slit 417 is pulled backward to allow the blade slit 353 of the blade plate 351 to cut the free end 137.

The manually ligature thread-cutting slit 418 is a slit adapted to manually cut the free end 137 of the ligature thread 130 by the surgeon if the benign prostatic hyperplasia surgical device 1 malfunctions during the surgery using the benign prostatic hyperplasia surgical device 1.

The needle manipulator-receiving groove 421 of the left frame 420 receives the needle manipulator 600 therein, and the trigger-rotating shaft 423 is adapted to rotatably fit a rotating shaft coupling tube 540 of the trigger 500 thereto. The lock releasing button-locking end 422 is adapted to allow a pusher lever hinge 645 of the pusher lever 640 to be elastically locked and supported thereon. As mentioned above, the sheath coupling member insertion groove 425 is adapted to fittedly couple the frame coupling plate 373 of the sheath cylinder 370 thereto. The indicator coupling shaft 427 is adapted to couple a frame coupling loop 741 of the thread indicator 740 of the ligature thread winder 700 thereto.

The pusher lever-rotating shaft coupling tube 428 is adapted to couple a pusher lever-rotating shaft 643 of the pusher lever 640 thereto so that the pusher lever 640 is rotatably supported.

The blade lock releasing lever 429 is rotatable to the left and right and thus comes into close contact with the locking wing 355a of the blade 350, while supporting the locking wing 355a, so that the blade-pressurizing button 355 is restrained in movement.

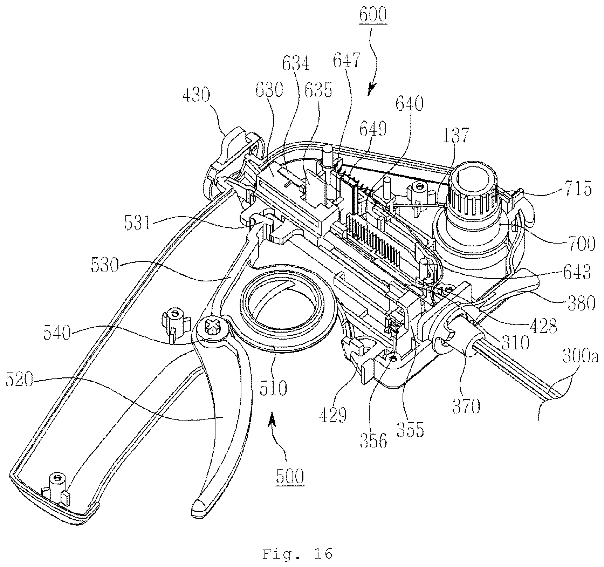

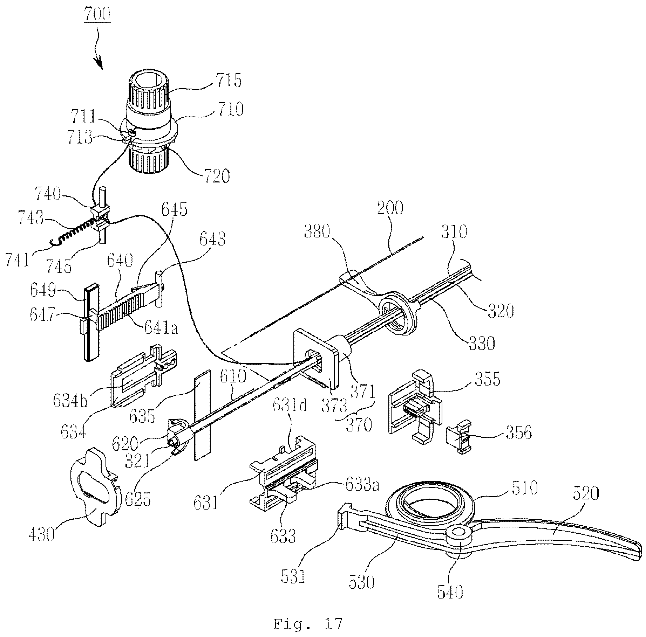

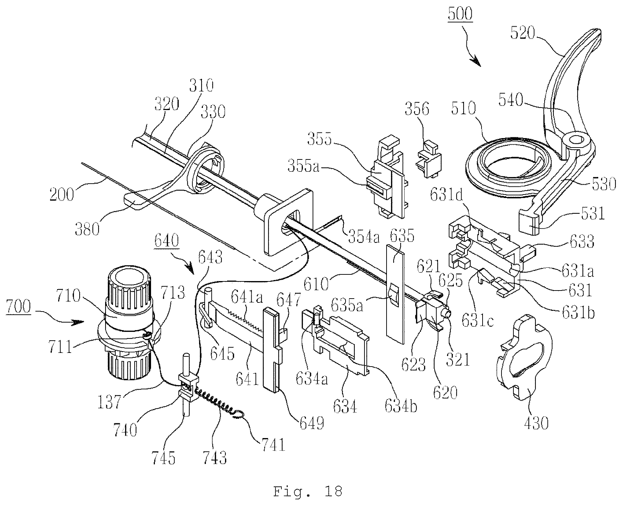

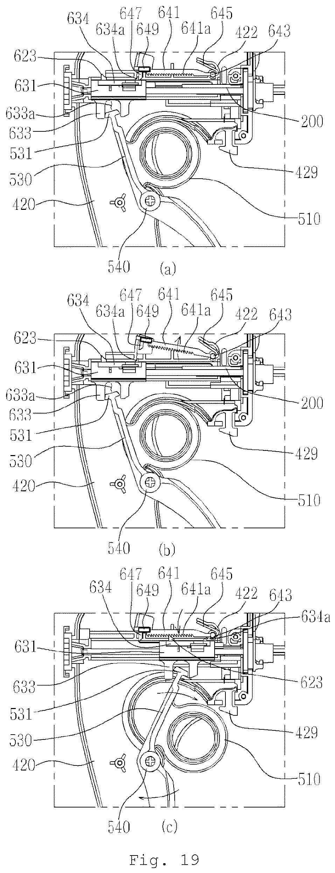

FIG. 16 is a perspective view showing the internal configuration of the handle frame 400, FIGs. 17 and 18 are exploded perspective views showing the sheath body 300a, the trigger 500, the needle manipulator 600, and the ligature thread winder 700, and FIG. 19 is an exemplary view showing a process of launching the needle 200.

The trigger 500 is coupled to the handle frame 400 in such a way as to be rotatable forward and backward, so that the trigger 500 is held by the surgeon's hand and transfers his or her manipulation to the needle manipulator 600, thereby allowing the needle 200 to be launched.

As shown in FIG. 17, the trigger 500 includes the loop 510 to which the surgeon's finger is fitted, a pulling lever 520 extending backward from the loop 510 in such a way as to be slant to a given angle, a pressurizing lever 530 extending upward from the pulling lever 520 in such a way as to be coupled to the needle holder 630, and the rotating shaft coupling tube 540 coupled to the trigger-rotating shaft 423 of the left frame 420.

As shown in FIG. 16, the trigger 500 is configured to allow the rotating shaft coupling tube 540 to be coupled to the trigger-rotating shaft 423, while allowing the loop 510 to be located in the trigger loop-receiving groove 411. Further, the pulling lever 520 is exposed to the outside from the handle frame 400, and the pressurizing lever 530 extends upward from the pulling lever 520 toward the upper portion of the handle frame 400 and is thus located between lever head coupling members 633 of the needle holder 630.

The pressurizing lever 530 has a coupling head 531 received in a lever head-receiving groove 633a formed between the lever head coupling members 633, and as shown in FIG. 19c, if the pulling lever 520 is pulled by the surgeon's hand, the pressurizing lever 530 rotates to the opposite direction to allow the needle holder 630 to move forward.

If the pulling lever 520 is pushed in the opposite direction to the pulling direction by the surgeon's hand, the pressurizing lever 530 is returned to its original state to allow the needle holder 630 to move backward.

As shown in FIG. 2, the surgeon's index finger is fitted to the loop 510 exposed to the outside from the handle frame 400, while the handle frame 400 is surrounded with his or her thumb, and next, the pulling lever 520 is held by the remaining fingers. The needle holder 630 is manipulated according to the pulling or pushing operation of the pulling lever 520.

The needle manipulator 600 is received in the handle frame 400 and moves according to the manipulation directions of the trigger 500 of the surgeon so that the needle 200 is launched to allow the anchor assembly 100 to be located on the prostate lobe A or B.

As shown in FIGs. 16, 17, and 18, the needle manipulator 600 includes the pusher 610 inserted into the rear end of the needle insertion tube 310, the pusher holder 620 fixedly coupled to the pusher 610 in such a way as to insert the rear end of the urethroscope insertion tube 320 thereinto, the needle holder 630 for receiving the pusher holder 620 therein in such a way as to be moved forward and backward by means of the manipulation of the trigger 500, and the pusher lever 640 located horizontally on top of the needle holder 630 to restrain the movement of the needle holder 630 and limit the moving distance thereof.

The pusher 610 has the shape of a rod with a given length. The pusher 610 extends from the front end of the pusher holder 620 to the given length capable of pressurizing the anchor assembly 100 and is thus inserted into the rear end of the needle insertion tube 310. The pusher 610 moves forward according to the forward movement of the needle holder 630 and physically pressurizes the anchor assembly 100 to allow the anchor assembly 100 located on the front end of the needle 200 to be discharged to the outside from the needle 200.

The pusher holder 620 is received in the needle holder 630 and moves together with the needle holder 630 to support the pusher 610 so that the pusher 610 pressurizes the anchor assembly 100. The pusher holder 620 has the shape of a rectangular box and has a urethroscope insertion tube-inserting hole 625 passing therethrough. An insertion tube rear end 321 of the urethroscope insertion tube 320 is inserted into the urethroscope insertion tube-inserting hole 625. The insertion tube rear end 321 is exposed to the outside from the rear side of the pusher holder 620 by a given length and serves to guide the urethroscope 800 inserted through the urethroscope-fixing member 430 toward the prostate lobe A or B.

The pusher holder 620 has a pair of holder hinges 621 disposed on both sides thereof. The holder hinges 621 are locked onto position-adjusting protrusions 631b and 631c protruding from the inner walls of the needle holder 630 when the pusher holder 620 moves inside the needle holder 630, thereby limiting the moving distance of the pusher holder 620.

The holder hinges 621 have the shapes of wings extending from both sides of the pusher holder 620 in such a way as to be elastically bent.

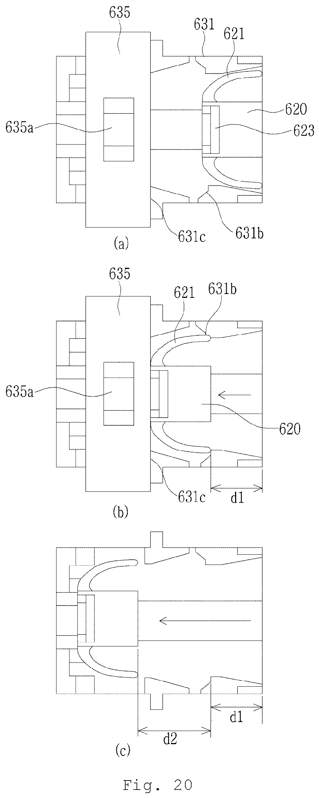

FIG. 20 is a plan view showing the process of moving the pusher holder 620 inside the needle holder 630. As shown in FIG. 20a, the first pusher holder position-adjusting protrusions 631b and the second pusher holder position-adjusting protrusions 631c slantly protrude from the inner walls of the needle holder 630 in a longitudinal direction of the needle holder 630.

In an initial position, the holder hinges 621 extending from both sides of the pusher holder 620 are brought into close contact with the inner walls of the needle holder 630, and if the pusher holder 620 moves forward inside the needle holder 630, as shown in FIG. 20b, the holder hinges 621 are elastically compressed, moved along the slant surfaces, and supportedly locked onto the first pusher holder position-adjusting protrusions 631b, so that the pusher holder 620 is limited in position.

After that, if the pusher block 635 blocking the path of the pusher holder 620 is removed by the surgeon to move the pusher holder 620 forward, the holder hinges 621 are elastically compressed again to softly move the pusher holder 620 and then supportedly locked onto the second pusher holder position-adjusting protrusions 631c, so that the pusher holder 620 is limited in position.

In this case, a distance d1 of the first pusher holder position-adjusting protrusions 631b and a distance d2 between the first pusher holder position-adjusting protrusions 631b and the second pusher holder position-adjusting protrusions 631c correspond to the lengths of the first anchor 110 and the second anchor 120, and accordingly, the needle 200 moves forward by the moving distance of the pusher 610 so that the first anchor 110 and the second anchor 120 are discharged.

Further, the pusher holder 620 has a position-adjusting tail 623 protruding from a top thereof. If the needle holder 630 moves forward by means of the trigger 500, the position-adjusting tail 623 is coupled to the pusher lever 640 to limit the moving speed and distance of the needle holder 630.

As shown in FIG. 19c, if the needle holder 630 moves forward by means of the operation of the trigger 500, the position-adjusting tail 623 engages with one of position-adjusting teeth 641a of the pusher lever 640 and moves, so that the needle holder 630 moves forward by a distance corresponding to one tooth size, without moving forward all at once.

The needle holder 630 moves forward and backward according to the operation of the trigger 500 by the surgeon to allow the pusher holder 620 and the needle 200 to be adjusted in position. The needle holder 630 includes a needle holder body 631 for movably receiving the pusher holder 620 therein, a needle holder cap 634 for covering a top of the needle holder body 631, and the pusher block 635 received therein to limit the moving distance of the pusher holder 620.

As shown in FIG. 18, the needle holder body 631 has a sheath-moving path 631a concavely formed in the middle region thereof in a longitudinal direction thereof. The sheath-moving path 631a receives the urethroscope insertion tube 320 fixed to the pusher holder 620 therein.

As mentioned above, the first pusher holder position-adjusting protrusions 631b and the second pusher holder position-adjusting protrusions 631c protrude from both inner walls of the needle holder body 631 and limit the moving position of the pusher holder 620.

The needle holder body 631 has a pusher block-receiving grooves 631d formed on both sides of a front portion thereof. As shown in FIG. 12, the pusher block-receiving grooves 631d receive the pusher block 635 therein. The pusher block 635 is received between the pusher block-receiving grooves 631d and the needle holder cap 634. Both ends of the pusher block 635 are exposed from both sides of the pusher block-receiving grooves 631d, and as shown in FIG. 2, they are thus exposed to the outside from the pusher block-moving slit 416 of the handle frame 400.

After the trigger 500 is primarily pulled by the surgeon, as shown in FIG. 20b, the pusher holder 620 moves up to the first pusher holder position-adjusting protrusions 631b where it is brought into close contact with the pusher block 635. As a result, the first anchor 110 of the anchor assembly 100 is discharged to the outside from the needle 200.

The pusher block 635 exposed to the outside from the pusher block-moving slit 416 is pressurized by the surgeon and thus separated from the interior of the handle frame 400. Further, the trigger 500 is secondarily pulled, and as shown in FIG. 20c, the pusher holder 620 moves forward to the maximum. As a result, the second anchor 120 is discharged to the outside from the needle 200.

The pair of lever head coupling members 633 is disposed on the underside of the needle holder body 631. The coupling head 531 of the pressurizing lever 530 of the trigger 500 is received between the lever head coupling members 633. The coupling head 531 is received in the lever head-receiving groove 633a between the lever head coupling members 633 and pressurizes the lever head coupling members 633 to the left and right according to the rotating directions of the trigger 500 so that the needle holder 630 is pressurized to be movable.

The needle holder cap 634 serves to cover the top of the needle holder body 631 to allow the pusher holder 620 and the pusher block 635 received therein to be fixed in position. The needle holder cap 634 has a lower locking protrusion 634a protruding upward from a front portion thereof. The lower locking protrusion 634a engages with an upper locking protrusion 647 of the pusher lever 640 to allow the needle holder 630 to be fixed in position, so that the needle holder 630 cannot move without the manipulation of the surgeon.

As shown in FIG. 18, the lower locking protrusion 634a has the sectional area of a right triangle and protrudes upward from the front portion of the needle holder cap 634. As shown in FIG. 17, the upper locking protrusion 647 protrudes downward from the rear portion of the pusher lever 640. The upper locking protrusion 647 has the sectional area of a right triangle positioned in the opposite direction to the lower locking protrusion 634a.

In an initial state, as shown in FIG. 19a, vertical surfaces of the lower locking protrusion 634a and the upper locking protrusion 647 with respect to each other are brought into close contact with each other, so that the forward movement of the needle holder 630 is limited.

If the pusher lever 640 rotates upward by the surgeon, as shown in FIG. 19b, the upper locking protrusion 647 is spaced apart from the lower locking protrusion 634a. In this case, if the trigger 500 is pulled by the surgeon, the needle holder 630 moves forward.

Further, as shown in FIG. 18, the needle holder cap 634 has a block protrusion-moving rail 634b with a given area formed to pass therethrough. The block protrusion-moving rail 634b receives a block protrusion 635a protruding from a top of the pusher block 635 therein. The block protrusion-moving rail 634b serves to limit the moving distance of the pusher block 635 if the needle holder 630 moves.

That is, as the block protrusion 635a moves by the distance of the block protrusion-moving rail 634b, the pusher block 635 is also limited in moving distance.

As mentioned above, the pusher block 635 serves to block the path of the pusher holder 620 to allow the pusher holder 620 to move by the distance d1 corresponding to the length of the first anchor 110 if the trigger 500 is primarily pulled by the surgeon. The pusher block 635 is removed from the surgical device 1 just before the trigger 500 is secondarily pulled by the surgeon. As described above, both ends of the pusher block 635 are exposed to the outside from the handle frame 400 through the pusher block-receiving groove 631d and the pusher block-moving slit 416, and the exposed pusher block 635 is pulled and removed by the surgeon.

As shown in FIG. 16, the pusher lever 640 is located horizontally on top of the needle holder 630 to restrain the movement of the needle holder 630 and limit the moving speed of the needle holder 630. As shown in FIGs. 17 and 18, the pusher lever 640 includes a horizontal pusher lever bar 641, the pusher lever-rotating shaft 643 located on the front portion of the pusher lever bar 641 to rotatably support the pusher lever bar 641, a pusher lever hinge 645 protruding inclined upward from the front portion of the pusher lever-rotating shaft 643 and applying an elastic force to the pusher lever bar 641 so that the pusher lever bar 641 rotating upward is returned to its initial position, the upper locking protrusion 647 protruding from the underside of the pusher lever bar 641, and the pusher lever button 649 extending from both sides of the rear portion of the pusher lever bar 641 by a given area and manipulated by the surgeon.

The pusher lever bar 641 has a given length and is located horizontally on top of the needle holder 630 inside the handle frame 400. As mentioned above, the position-adjusting teeth 641a are formed on the underside of the pusher lever bar 641 in a longitudinal direction of the pusher lever bar 641, and if they come into contact with the position-adjusting tail 623 of the pusher holder 620, they thus limit the moving position of the needle holder 630. The needle holder 630 moves forward and backward only by the length of the position-adjusting tail 623 formed.

If the trigger 500 is pulled by the surgeon, as shown in FIG. 19c, the needle holder 630 moves forward along the position-adjusting tail 623.

The pusher lever-rotating shaft 643 is coupled to the pusher lever-rotating shaft coupling tube 428 of the left frame 420 to allow the pusher lever bar 641 to rotate upward and downward. The pusher lever hinge 645 extends from the pusher lever-rotating shaft 643 in such a way as to be inclined upward by a given angle. The pusher lever hinge 645 is fitted between the pusher lever-rotating shaft 643 and the lock releasing button-locking end 422 of the left frame 420 if the pusher lever-rotating shaft 643 is fitted to the pusher lever-rotating shaft-coupling tube 428.

As shown in FIG. 19a, the pusher lever hinge 645 is brought into close contact with the inner wall surface of the lock releasing button-locking end 422. In the above state, as shown in FIG. 19b, if the pusher lever button 649 is pressurized upward by the surgeon to rotate the pusher lever bar 641 around the pusher lever-rotating shaft 643, the pusher lever hinge 645 is compressed between the pusher lever-rotating shaft 643 and the lock releasing button-locking end 422.

As shown in FIG. 19c, if the pusher lever button 649 is released by the surgeon, the compressed pusher lever hinge 645 applies the elastic force to the pusher lever bar 641 so that the pusher lever bar 641 rotates and is elastically returned to a horizontal level.

As mentioned above, the upper locking protrusion 647 engages with the lower locking protrusion 634a of the needle holder cap 634 to restrain the movement of the needle holder 630.

The pusher lever button 649 extends horizontally to the left and right sides from a top of the rear portion of the pusher lever bar 641 and is thus received in the pusher lever button-receiving groove 414 of the handle frame 400. As shown in FIG. 2, the pusher lever button-receiving groove 414 has a given area extending upward and downward, and therefore, the pusher lever button 649 exposed to the outside from the pusher lever button-receiving groove 414 is pressurized upward to lift up the pusher lever bar 641.

As shown in FIG. 19a, the lower locking protrusion 634a and the upper locking protrusion 647 engage with each other in a state where the pusher lever button 649 moves down, so that the movement of the needle holder 630 is limited. In the above state, as shown in FIG. 19b, the pusher lever button 649 exposed to the outside from the handle frame 400 is lifted up by the surgeon, so that the pusher lever bar 641 rotates upward. As a result, the lower locking protrusion 634a and the upper locking protrusion 647 are spaced apart from each other, and as shown in FIG. 19c, the trigger 500 is pulled by the surgeon to allow the needle holder 630 to move forward.

The ligature thread winder 700 serves to allow the free end 137 of the ligature thread 130 to be wound thereon by the surgeon so that the length of the free end 137 is adjusted to allow the compression intensity of the ligature thread 130 against the prostate lobe A or B to be controlled. As shown in FIG. 12, the ligature thread winder 700 includes the winder shaft 710, one-way rotation-limiting teeth 720 formed on the winder shaft 710 to limit the rotational direction of the winder shaft 710 so that the winder shaft 710 rotates only in one direction, a thread winder lock releasing button 730 located on a top of the left frame 420 to restrain the rotation of the winder shaft 710, and the thread indicator 740 located on the moving path of the ligature thread 130 between the winder shaft 710 and the needle 200 to sense the tension of the free end 137.

The winder shaft 710 is received in the thread winder-receiving groove 412 of the left frame 420 and the right frame 410. In this case, rotation manipulation knobs 715 located on both ends of the winder shaft 710 are exposed to the outside from the thread winder-receiving groove 412 and held and rotated by the surgeon's hand.

As shown in FIG. 17, the winder shaft 710 has a thread-fitting loop 711 located on the outer peripheral surface thereof to fix the ligature thread 130 thereto. The ligature thread 130 is fixedly knotted to the thread-fitting loop 711 and then wound multiple times on the outer peripheral surface of the winder shaft 710.

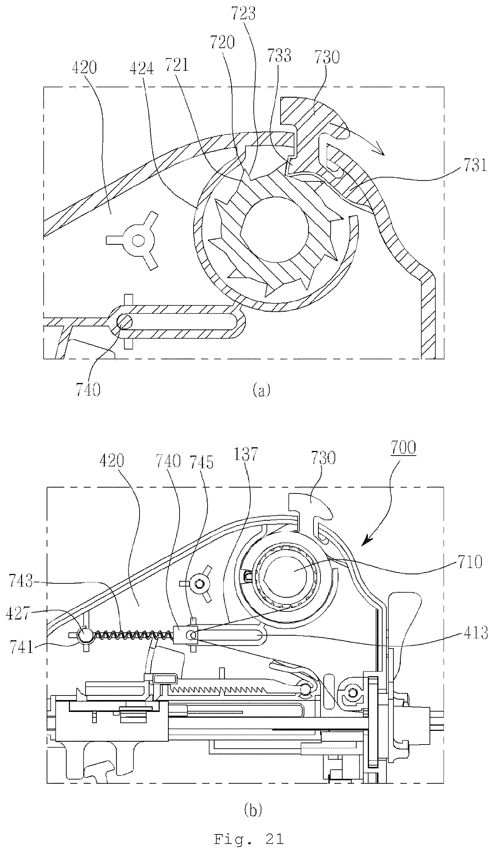

FIG. 21a is an exemplary view showing an operating process of the thread winder lock releasing button 730 and the one-way rotation-limiting teeth 720. The one-way rotation-limiting teeth 720 protrude from the outer peripheral surface of the winder shaft 710 at given intervals. In this case, each of the one-way rotation-limiting teeth 720 has slant surfaces 721 on one surface thereof and vertical surfaces 723 on the other surface thereof.

The thread winder lock releasing button 730 has a frame connection end 731 on one side of an underside thereof in such a way as to be fixed to the handle frame 400 and top exposed to the outside of the handle frame 400. A teeth lock protrusion 733 protrudes vertically from the other side of the underside thereof facing the frame connection end 731. An underside of the thread winder lock releasing button 730 between the frame connection end 731 and the teeth lock protrusion 733 is a slowly curved surface.

In an initial state, the teeth lock protrusion 733 is kept to a contacted state with the vertical surface 723 of any one of the one-way rotation-limiting teeth 720. The thread winder lock releasing button 730 is pulled in a clockwise direction and the rotation manipulation knobs 715 are rotated by the surgeon, so that the ligature thread 130 is wound.

In this case, the winder shaft 710 is rotatable only in a direction along which the ligature thread 130 is wound. That is, the winder shaft 710 rotates only in a counterclockwise direction relative to FIG. 21a. If the winder shaft 710 rotates in the counterclockwise direction, the slant surface 721 of any one of the one-way rotation-limiting teeth 720 comes into contact with the curved surface of the thread winder lock releasing button 730, so that the winder shaft 710 can rotate. Contrarily, if the winder shaft 710 rotates in the clockwise direction, the vertical surface 723 of any one of the one-way rotation-limiting teeth 720 comes into contact with the teeth lock protrusion 733, so that the rotation of the winder shaft 710 is limited.

The thread indicator 740 serves to indicate the current tension of the free end 137 of the ligature thread 130 to the surgeon. The thread indicator 740 has indication protrusions 745 protruding outward from both sides thereof. As shown in FIG. 2, the indication protrusions 745 are movable to the left and right along the indicator-receiving groove 413 of the right frame 410.

The thread indictor 740 has a coil spring type elastic member 743 with a given length coupled thereto, and as shown in FIG. 11, the frame coupling loop 741 coupled to the front end of the elastic member 743 is fixed to the indicator coupling shaft 427 of the left frame 420.

As shown in FIG. 16, the ligature thread 130 extends from the winder shaft 710, passes through the thread indicator 740 and the needle insertion tube 310, and is then coupled to the first anchor 110 and the second anchor 120. If the winder shaft 710 is rotated to wind the ligature thread 130 thereon by the surgeon, the tension of the ligature thread 130 increases so that the thread indicator 740 gradually moves to the right. The current position of the thread indicator 740 is checked during the rotation of the rotation manipulation knobs 715 by the surgeon, and if the ligature thread 130 reaches desired tension, the free end 137 is cut by means of the blade 350.

Next, a benign prostatic hyperplasia surgical process using the benign prostatic hyperplasia surgical device 1 according to the embodiment of the present invention will be explained with reference to FIGs. 2 to 24.

FIGs. 23 and 24 are exemplary views showing a benign prostatic hyperplasia surgical process according to the present invention.

As shown in FIG. 2, the benign prostatic hyperplasia surgical device 1 is prepared in a sterilized and packed state with the components completely put together.

In this case, as shown by the enlarged portion of FIG. 10, the anchor assembly 100 is fitted to the needle slot 220 of the needle 200, and the ligature thread 130 is exposed to the outside from the outer needle exposure hole 331 of the blade plate 351 and received in the suture arranger 360.

As shown in FIG. 10, next, the free end 137 of the ligature thread 130 is exposed to the outside from the rear end of the needle insertion tube 310, passes through the thread indicator 740, and is then wound on the outer peripheral surface of the winder shaft 710 of the ligature thread winder 700.

In a preparation state before surgery starts, as shown in FIG. 19a, the lower locking protrusion 634a of the needle holder 630 is located to engage with the upper locking protrusion 647 of the pusher lever 640, thereby limiting the movement of the needle holder 630.

As the movement of the needle holder 630 is limited, the coupling head 531 of the pressurizing lever 530 of the trigger 500 is inserted between the lever head coupling members 633 of the needle holder 630, so that the trigger 500 is fixed in position.

Local anesthesia for an interior of the urethra is performed using anesthetic jelly by the surgeon, and otherwise, spinal anesthesia or general anesthesia for the patient is performed. As shown in FIG. 23a, the urethra C is in a closed state because of enlarged left and right prostatic lobes A and B.

The outer sheath S is first inserted into the urethral meatus by the surgeon, and the inserted state of the outer sheath S is kept until the surgery is completed.

The suture arranger 360 is removed from the needle-receiving sheath 300 by the surgeon, and as shown in FIG. 23b, the needle-receiving sheath 300 is inserted into the urethra C through the outer sheath S inserted. In this case, the needle 200 is in a state of being received in the needle insertion tube 310.

As shown in FIG. 19b, the pusher lever button 649 exposed to the outside from the handle frame 400 is pressurized upward by the surgeon. As the pusher lever button 649 is pressurized upward, the needle holder 630 and the trigger 500 can move.

In the above state, the interior of the urethra C is inspected by the surgeon through the urethroscope 800 inserted into the needle-receiving sheath 300, thereby checking positions where the anchors 110 and 120 will be located. The positions where the anchors 110 and 120 will be located are obtained with anatomical points such as the bladder neck, the verumontanum, and the like.

The needle-receiving sheath 300 is tilted at an angle of 20 degrees or more toward the left prostate lobe. This is just exemplary, and the surgeon can determine whether he or she performs the surgery for the left prostate lobe or the right prostate lobe first according to his or her independent judgment.

Furthermore, as shown in FIG. 19c, the pulling lever 520 of the trigger 500 is pulled manually by the surgeon to rotate the pressurizing lever 530, thereby moving the needle holder 630 forward. As a result, the needle 200 is launched.

The pulling lever 520 is pulled by the surgeon until the needle holder 630 moves forward to the maximum or until the needle 200 does not move forward anymore.

While the needle holder 630 is moving forward, the position-adjusting tail 623 moves along the position-adjustment teeth 641a of the pusher lever bar 641. Further, the pusher holder 620 moves inside the needle holder 630, together with the needle holder 630, and as shown in FIG. 20b, the pusher holder moves until it comes into close contact with the pusher block 635 or until the holder hinge 621 is locked onto the first pusher holder position-adjusting protrusion 631b.

If the needle holder 630 moves forward by a given distance, the surgeon's hand which pressurizes the pusher lever button 640 upward is released. If released, the pusher lever 640 is returned to its initial position by means of the elastic force of the pusher lever hinge 645.

The needle 200 is launched by means of the forward movements of the needle holder 630 and the pusher holder 620, and as shown in FIG. 23c, the needle 200 is launched to the outside from the needle guide member 340, penetrates the prostate lobe A, and is put on the outer side of the prostate lobe.

In the above state, as shown in FIG. 9a, the first anchor 110 and the second anchor 120 are received sequentially in the needle slot 220, and the pusher 610 is inserted into the rear end of the needle 200.

In the above state, the loop 510 of the trigger 500 is pressurized by the surgeon, whereas the pusher lever button 649 is not pressurized upward again by him or her, thereby allowing the needle holder 630 moved forward to move backward. In this case, as the position-adjusting tail 623 is locked onto the position-adjusting teeth of the pusher lever 640, the pusher holder 620 is fixed in position, and therefore, only the needle holder 630 moves backward. As the pusher 610 is fixed in position and only the needle 200 moves backward by means of the needle holder 630, as shown in FIG. 9b, the first anchor 110 is discharged to the outside from the needle 200.

As shown in FIG. 23d, the first anchor 110 discharged to the outside from the needle 200 is located on the outer side of an upper portion of the left prostate lobe A.

If the discharge of the first anchor 110 is completed, the pusher lever button 649 is pressurized upward, and the loop 510 of the trigger 500 is pressurized, so that the needle holder 630 moves backward to the maximum. In this case, the pusher holder 620 is fixed in position inside the needle holder 630 by means of the pusher block 635. As a result, the pusher 610 and the needle 200 move backward together.

The needle-receiving sheath 300 moves in position to a position where the second anchor 120 will be discharged. Next, the pusher block 635 is pulled and removed from the handle frame 400. After that, the above-mentioned processes are repeatedly performed so that the pusher lever button 649 is pressurized upward and the pulling lever 520 is pulled, thereby moving the needle holder 630 forward. As a result, the needle 200 penetrates a lower portion of the left prostate lobe A. In the above state, the loop 510 of the trigger 500 is pressurized by the surgeon, whereas the pusher lever button 649 is not pressurized upward by him or her, thereby allowing the needle holder 630 moved forward to move backward.

In this case, as shown in FIG. 20C, since the pusher block 635 is removed, the pusher holder 620 moves inside the needle holder 630 by the distance d2 capable of pressurizing the second anchor 120. As the pusher holder 620 and the pusher 610 are fixed in position by means of the pusher lever 640 and only the needle 200 moves backward by means of the needle holder 630, the second anchor 120 is discharged to the outside from the needle 200. If the discharge of the second anchor 120 is completed, the pusher lever button 649 is pressurized upward, and the loop 510 of the trigger 500 is pressurized, so that the needle holder 630 moves backward to the maximum.

As shown in FIG. 24a, the second anchor 120 discharged to the outside from the needle 200 is located on the outer side of the lower portion of the left prostate lobe A. In this case, the first anchor 110 and the second anchor 120 are in a state of being connected to each other by means of the ligature thread 130, and the free end 137 is wound on the ligature thread winder 700 through the needle insertion tube 310.

In the state where the first anchor 110 and the second anchor 120 are placed on the outer side of the prostate lobe A, as shown in FIG. 24b, the length-adjustable loop 133 is not adjusted in length, and thus, the ligature thread 130 is positioned loosely, so that the prostatic tissue is not compressed at all.

The inside is inspected with the urethroscope by the surgeon, and as shown in FIG. 21a, the thread winder lock releasing button 730 is pulled to allow the teeth lock protrusion 733 to be spaced apart from the vertical surface 723 of any one of the one-way rotation-limiting teeth 720. Next, the rotation manipulation knobs 715 are rotated to allow the free end 137 to be wound on the winder shaft 710.

If the free end 237 is wound on the winder shaft 710, the length-adjustable loop 133 gets shorter to allow the left prostate lobe A to be compressed against the anchor assembly 100. As shown in FIG. 21b, the scale 413a indicated by the thread indicator 740 is checked by the surgeon so that the tension of the free end 137 is checked.

If the tension of the free end 137 indicated by the scale 413a has been checked and a degree of opening for the urethra C through the compression of the anchor assembly 100 against the left prostate lobe A has been checked by means of the urethroscope 800, as shown in FIG. 22b, the blade lock releasing lever 429 is pressurized to rotate by the surgeon. If the locked state between the blade lock releasing lever 429 and the locking wing 355a of the blade-pressurizing button 355 is released, the blade-pressurizing button 355 is pushed backward.

As a result, as shown in FIG. 15b, the blade plate 351 slides toward the rear portion of the inner needle exposure hole 352, and the free end 137 of the ligature thread 130 is caught in the blade slit 353 of the blade plate 351 and then cut.

The cut free end 137 of the ligature thread 130 is 1 mm or less in length and thus buried in the prostatic tissue. Therefore, as shown in FIG. 24c, the surgery for opening the urethra through the compression of the anchor assembly 100 against the left prostate lobe A is completed.

Next, the needle-receiving sheath 300 is removed out of the urethra by the surgeon, and an anchor assembly 100 of a new benign prostatic hyperplasia surgical device 1 is located on the right prostate lobe B in the same way as the left prostate lobe A.

As a result, as shown in FIG. 24d, the pair of the anchor assemblies 100 is located at the left prostate lobe A and the right prostate lobe B to open the urethra C. In this case, each anchor assembly 100 continuously compresses the prostatic tissue in the form of a "⊏" character or oval connecting the first anchor 110, the second anchor 120, and the ligature thread 130 with one another. Therefore, the length ℓ of the urethra C opened under the compression of the ligature thread 130 is adjustable, and further, an opening width d of the urethra C compressed can be adjusted.

As described above, when compared with the conventional surgical method for compressing a portion of the prostatic tissue, the benign prostatic hyperplasia surgical device according to the present invention can increase urine flow rate since the prostatic tissue compressed against the ligature thread and the pair of the anchors is continuous, and it is more effective for patients who have a large prostate gland.

Moreover, the benign prostatic hyperplasia surgical device according to the present invention can adjust the length of the ligature thread by winding the free end of the ligature thread, so that compression intensity against the prostate gland can be adjusted to a degree desired by the surgeon.

Therefore, the benign prostatic hyperplasia surgical device according to the present invention can adjust the compression intensity against the prostate gland according to various symptoms of the benign prostatic hyperplasia presented by patient.

The surgical method using the benign prostatic hyperplasia surgical device according to the present invention does not need general anesthesia or long operation time, unlike laser surgery or electrical surgery using thermal energy in which general anesthesia or long operation time is required.

Additionally, the benign prostatic hyperplasia surgical device according to the present invention can make quick operation time and local anesthesia possible through the simple anchor installation method, and have no side effects, such as retrograde ejaculation, impotence or hematuria, since the prostatic tissue is not cut. In addition, the surgical method using the benign prostatic hyperplasia surgical device according to the present invention is minimally invasive, and therefore, if the surgery is ineffective, a patient can have another surgery any time.

In the above, the technical ideas of the present invention have been described.

It is to be appreciated that those skilled in the art can change or modify the embodiments from the above description in various ways. Even if it is not clearly illustrated or described herein, it is to be appreciated that those skilled in the art can change or modify the embodiments from the above description in various ways without departing from the scope and spirit of the present invention, and such changes and modifications belong to the scope of the present invention. While the present invention has been described with reference to the particular illustrative embodiments, it is not to be restricted by the embodiments but only by the appended claims.

## Claims

1. A benign prostatic hyperplasia surgical device comprising:
a plurality of anchors (110 and 120) disposed on the prostate gland compressing the urethra;
a ligature thread (130) connecting the plurality of anchors (110 and 120) to each other so that the plurality of anchors (110 and 120) continuously compress prostatic tissue to allow the urethra to be open;
an elastic needle (200) receiving the plurality of anchors (110 and 120) therein and inserted into the urethra to guide the plurality of anchors (110 and 120) and the ligature thread (130) so that the plurality of anchors (110 and 120) and the ligature thread (130) are located on the prostate gland; and
a needle launching means for launching the needle (200) so that the needle (200) is inserted into the urethra to allow the plurality of anchors (110 and 120) and the ligature thread (130) to be discharged through the needle (200).

2. The benign prostatic hyperplasia surgical device according to claim 1, wherein if the plurality of anchors (110 and 120) consists of a pair of a first anchor (110) and a second anchor (120), the first anchor (110) is fixed to the outer side of an upper portion of the prostate gland, whereas the second anchor (120) is fixed to the outer side of a lower portion of the prostate gland, and the ligature thread (130) comprises a fixed end (131) fixed to the first anchor (110), a length-adjustable loop (133) coupled to the second anchor (120) in such a way as to be adjustable in length, a slip knot (135) for connecting the fixed end (131) and the length-adjustable loop (133) to each other, and a free end (137) extending from the length-adjustable loop (133).

3. The benign prostatic hyperplasia surgical device according to claim 1, wherein if the plurality of anchors (110 and 120) consists of a pair of a first anchor (110) and a second anchor (120), the first anchor (110) is fixed to the outer side of an upper portion of the prostate gland, whereas the second anchor (120) is fixed to the outer side of a lower portion of the prostate gland, and the ligature thread (130) comprises a length-adjustable loop (133) coupled to the first anchor (110) and the second anchor (120) in such a way as to be adjustable in length and a slip knot (135) coupled to a free end (137) and a fixed end (131) of the length-adjustable loop (133) in such a way as to support the length-adjustable loop (133) to allow the length-adjustable loop (133) to be adjustable in length.

4. The benign prostatic hyperplasia surgical device according to claim 1, wherein if the plurality of anchors (110 and 120) consists of a pair of a first anchor (110) and a second anchor (120), the first anchor (110) is fixed to the outer side of an upper portion of the prostate gland, whereas the second anchor (120) is fixed to the outer side of a lower portion of the prostate gland, and the ligature thread (130) comprises a fixing knot (131a) fixed to the first anchor (110), a free end (137) extending from the fixing knot (131a) and passing through the second anchor (120), and a lock anchor (150) coupled to the free end (137) at the inner side of the lower portion of the prostate gland in such a way as to fix the free end (137) in position.

5. The benign prostatic hyperplasia surgical device according to claim 1, wherein if the plurality of anchors (110 and 120) consists of a first anchor (110), a second anchor (120), and a third anchor (140), the first anchor (110) is fixed to the outer side of an upper portion of the prostate gland, the second anchor (120) is fixed to the outer side of a central portion of the prostate gland, and the third anchor (140) is fixed to the outer side of a lower portion of the prostate gland, the ligature thread (130) comprising a fixed end (131) fixed to the first anchor (110), a free end (137) extending from the fixed end (131) and passing through the second anchor (120) and the third anchor (140), and a lock anchor (150) coupled to the free end (137) at the inner side of the lower portion of the prostate gland in such a way as to fix the free end (137) in position.

6. The benign prostatic hyperplasia surgical device according to claim 1, wherein if the plurality of anchors (110 and 120) consists of a pair of a first anchor (110) and a second anchor (120), the first anchor (110) is fixed to the outer side of a central portion of the prostate gland, and the second anchor (120) is fixed to the inner side of the central portion of the prostate gland in such a way as to face the first anchor (110).

7. The benign prostatic hyperplasia surgical device according to any one of claims 1 to 6, wherein the needle launching means comprises:
a needle-receiving sheath (300) adapted to receive the needle (200) therein;
a needle manipulator (600) for manipulating the movement of the needle (200) so that the needle (200) is discharged through the end of the needle-receiving sheath (300);
a trigger (500) configured to be held to manipulate the needle manipulator (600) so that the needle (200) is launched; and
a handle frame (400) for supporting the rear end of the needle-receiving sheath (300), the needle manipulator (600), and the trigger (500) thereagainst.
